# EUROPEAN PATENT SPECIFICATION

(11) **EP 3 193 895 B1**
(45) Date of publication and mention of the grant of the patent: **15.04.2020**
(21) Application number: 14799963.5
(22) Date of filing: 31.10.2014
(51) Int. Cl.: A61K 35/747, A61P 3/06, A61P 31/04

(54) **LACTOBACILLUS PLANTARUM INDUCIA STRAIN DSM 21379 AS HYPOCHOLESTEROLEMIC AGENT AND ANTIMICROBIAL AGENT AGAINST CLOSTRIDIUM DIFFICILE**
LACTOBACILLUS PLANTARUM INDUCIA-STAMM DSM 21379 ALS HYPOCHOLESTERINÄMISCHES MITTEL UND ANTIMIKROBIELLES MITTEL GEGEN CLOSTRIDIUM DIFFICILE
SOUCHE INDUCIA DSM 21379 DE LACTOBACILLUS PLANTARUM UTILISÉE COMME AGENT HYPOCHOLESTÉROLÉMIQUE ET AGENT ANTIMICROBIEN DIRIGÉ CONTRE CLOSTRIDIUM DIFFICILE

(30) Priority: 03.04.2014 US 201414244284
(43) Date of publication of application: 26.07.2017
(73) Proprietor: BioCC OÜ, 51014 Tartu (EE)
(72) Inventor: MIKELSAAR, Marika, EE-51005 Tartu (EE); SONGISEPP, Epp, EE-50107 Tartu (EE); RÄTSEP, Merle, EE-50109 Tartu (EE); HÜTE, Pirje, EE-50110 Tartu (EE); SMIDT, Imbi, EE-61622 Tartumaa (EE); TRUUSALU, Kai, EE-50107 Tartu (EE); SEPP, Epp, EE-10119 Tallinn (EE); MIKELSAAR, Raik-Hiio, EE-51005 Tartu (EE); KILK, Kalle, EE-50404 Tartu (EE); STSEPETOVA, Jelena, EE-50707 Tartu (US); KÕLJALG, Siiri, EE-50404 Tartu (EE); VALLAS, Mirjam, EE-50408 Tartu (EE)
(74) Representative: Kahu, Sirje
(86) International application number: PCT/EE2014/000007
(87) International publication number: WO 2015/149818

(56) References cited:
- WO-A1-2009/138092
- WO-A1-2011/042333
- DE VRIES M C ET AL: "Lactobacillus plantarum-survival, functional and potential probiotic properties in the human intestinal tract", INTERNATIONAL DAIRY JOURNAL, ELSEVIER APPLIED SCIENCE, BARKING, GB, vol. 16, no. 9, 1 September 2006 (2006-09-01), pages 1018-1028, XP024963305, ISSN: 0958-6946, DOI: 10.1016/J.IDAIRYJ.2005.09.003 [retrieved on 2006-09-01]
- P. NAABER: "Inhibition of Clostridium difficile strains by intestinal Lactobacillus species", JOURNAL OF MEDICAL MICROBIOLOGY, vol. 53, no. 6, 1 June 2004 (2004-06-01), pages 551-554, XP055161877, ISSN: 0022-2615, DOI: 10.1099/jmm.0.45595-0

## Description

### TECHNICAL FIELD

The present invention relates to the field of microbiology and probiotics refering to *Lactobacillus plantarum* Inducia (DSM21379) strain for preventing cholesterol metabolism disorders and cardiovascular disorders.

### BACKGROUND ART

### LDL-cholesterol

One of the factors leading to cardiovascular disease (CVD) is abnormally elevated cholesterol level. Recently, the view of high cholesterol as damaging agent has been reverted to abnormality of its particles, particularly low density lipoprotein - cholesterol (LDL-c) and Apolipoprotein B (ApoB). LDL- c accounts 60 - 70% of total cholesterol. LDL- c particles carry cholesterol, triglycerides, fat-soluble vitamins and antioxidants. The LDL-cholesterol is an important modulator for prevention of atherosclerosis and maintenance of cardiovascular health. Oxidized low-density lipoprotein (Ox-LDL) is a marker of oxidative stress specific to LDL particles. Ox-LDL could be considered one of biochemical risk markers for coronary heart diseases. Thus, the LDL-c is widely recognized as an established cardiovascular risk marker according to results of numerous clinical trials that demonstrate the ability of LDL-c to independently cause development and progression of coronary heart disease (http://www.mayomedicallaboratories.com/articles/communique/2011/11.html, retrieved 14.03.2014).
Bile salt hydrolase (BSH) activity of probiotics has been considered one of the main mechanisms of hypocholesterolemic effect (Pereira, D.I., McCartney, A.L., Gibson, G.R An in vitro study of the probiotic potential of a bile-salt-hydrolyzing Lactobacillus fermentum strain, and determination of its cholesterol-lowering properties. Appl Environ Microbiol. 2003: 69: 4743-4752; Liong, M.T., Shah, N.P. Bile salt deconjugation ability, bile salt hydrolase activity and cholesterol co-precipitation ability of lactobacilli strains. Int Dairy J. 2005: 15: 391-398; Lye, H.S., Rahmat-Ali, G.R., Liong, M.T. Mechanisms of cholesterol removal by lactobacilli under conditions that mimic the human gastrointestinal tract, Int Dairy J. 2010: 20 (3): 169-175). BSH (EC 3.5.1.24; cholylglycine hydrolase) is an enzyme that catalyses the hydrolysis of glycine- and/or taurineconjugated bile salts into amino acid residues and free bile acids. The BSH activity in lactic acid bacteria is strongly correlated with natural habitat and is mainly observed in species associated with the gastrointestinal tract incl. *Lactobacillus sp.* However, wide variations exist among strains.

The close relation between mucosal epithelial cells of host gut and microbiota is of utmost importance for health. Among indigenous microbiota of gastrointestinal tract (GIT) the lactic acid bacteria assimilate cholesterol from dietary products (Gilliland, S. E., Nelson, C. R., Maxwell, C., Assimilation of cholesterol by Lactobacillus acidophilus. Appl Environ Microbiol. 1985: 49, 377-381). LDL-lowering effects of *Lactobacillus plantarum* strains due to their high bile salt hydrolase (BSH) were disclosed in the patent appplication and corresponding patent of Cuñé Castellana, 2009 (WO 2011/042333 A1, EP2485743B1; AB Probiotics S.A.). The described *Lactobacillus plantarum* strains CECT 7528, CECT 7526 and CECT 7529 as single or in composition demonstrated both *in vitro* and *in vivo* the cholesterol lowering ability. These strains have bile salt hydrolases (BHS) activity, also the antagonistic activity to inhibit the growth of pathogenic strains (*Salmonella enterica* Enteritidis, *Salmonella enterica* Typhimurium, *Yersinia pseudotuberculosis, Clostridium perfringens, Clostridium ramnosus, Enterococcus faecalis*) and can be used as probiotic bacteria. Pereira *et al.* (Pereira, D. I., McCartney, A. L., Gibson, G. R., An in vitro study of the probiotic potential of a bile-salt-hydrolyzing Lactobacillus fermentum strain, and determination of its cholesterol-lowering properties. Appl Environ Microbiol. 2003: 69, 4743-4752) have demonstrated the role of short-chain fatty acid concentrations, specifically the molar proportion of propionate and/or bile salt deconjugation as the major mechanism involved in the purported cholesterol-lowering properties of *L. fermentum.*
However, the effect of *Lactobacillus* spp strains on levels of serum cholesterol (and cholesterol fractions) is strain-specific and dependant on the origin and properties of a certain strain (Tanaka, H., K. Doesburg, T. Iwasaki and I. Mireau, Screening of lactic acid bacteria for bile salt hydrolase activity. J. Dairy Sci. 1999: 82: 2530-2535).

### Probiotic

A probiotic is a viable microbial food component which has a demonstrated benefit on human health when given in appropriate amounts (Joint FAO/WHO Working Group on Drafting Guidelines for the Evaluation of Probiotics in Food ftp://ftp.fao.org/docrep/fao/009/a0512e/a0512e00.pdf Retrieved 14.03.2014).

The narrower definition accepts probiotics as living microorganisms that beneficially affect the host by improving the intestinal microbial balance. Currently a probiotic product is a strain-specific preparation targeting several host functions (anti-infectious, morphologic, immunologic, metabolic) in order to improve health by either supporting host physiologic activity or by reducing the risk of disease. It has been generally accepted that the probiotic potential of different strains of the same species may have different probiotic effects (Ouwehand, A. C., Salminen, S., Isolauri, E., Probiotics: an overview of beneficial effects. Antonie Van Leeuwenhoek. 2002: 82, 279-289; Ljungh, A., Wadström, T., Lactic acid bacteria as probiotics. Curr Issues Intest Microbiol. 2006: 7, 73-89).

Most widely applied probiotics belong to species of *Bifidobacteria* and *Lactobacillus.* Probiotics are commonly consumed as part of fermented foods e.g. yoghurt, kefir, cheese or dietary supplements. The colonic microbiota is well stabilised and due to mucosal microbiota it does not change easily. However, if given in sufficient amount (daily dose at least 10⁹ CFU) it is possible to demonstrate the effect of probiotic intervention.

The therapeutic concept with probiotics is to reduce or eliminate potential pathogens and toxins, to release nutrients, antioxidants, growth factors, to stimulate the gut motility and modulate innate and adaptive immunity.

*Lactobacillus plantarum* is widely spread in nature from fermented plant material, dairy and meat products, and also in GIT of humans (Hammes, W.P., Weiss, N. and Hertel, K. The genera Lactobacillus and Carnobacterium. Prokaryotes. 2006: 4:320-403). Strains of *L. plantarum* species are available as probiotic food or supplement (L. *plantarum* 299v, DSM 9843, Probi AB, Sweden) and belong to multispecies probiotic supplement VSL#3 (VSL Pharmaceuticals, Inc. USA).

The strain *Lactobacillus plantarum* Inducia (DSM 21379) has been disclosed as a probiotic that enhances natural cellular immunity and barrier function of gut mucosa via secretion of antimicrobial peptide plantaricin, induction of cytokine IL-6, production of putrescine and increases the amount of lymphatic tissue in intestine (Mikelsaar et al., WO2009/138092A1 and EP2288360B1, 2009, Bio-Competence Centre of Healthy Dairy Products).

### Disturbed microbial ecology of gut

It is well known that the application of broad-spectrum antimicrobial preparations for treatment of infections and inflammatory complications may cause profound imbalance among GI microbiota.

### Clostridium difficile infection

*Clostridium difficile* was identified in the 1970's as the causative agent of antibiotic associated diarrhoea. The anaerobic spore-forming intestinal pathogen *Clostridium difficile* is spread in hospitals and elderly homes (Britton, R. A., Young, V. B., Interaction between the intestinal microbiota and host in Clostridium difficile colonization resistance. Trends Microbiol. 2012: 20, 313-319). *C. difficile* infection of exogenous origin is initiated by infection with the spore form of *C. difficile. C. difficile* elicits disease through the actions of secreted toxins, which are produced by vegetative cells, not by spores. Endospore production is vital for the spread of *Clostridium difficile* infection. In order to cause disease, these spores must germinate and return to vegetative cell growth (Burns D.A, Heap J.T. Minton N.P. Clostridium difficile spore germination: an update. Res Microbiol. 2010;161(9):730-4).
In a quarter of patients (25%) infected with *C. difficile* develop serious sequela such as pseudomembraneous colitis (PMC). *Clostridium difficile-associated* diarrhea (CDAD) increases mortality rates, lengthens hospitalization and dramatically increases overall health care costs. The treatment guidelines to date stress the use of vancomycin in moderate and severe pseudomembraneous colitis (http://www.uptodate.com/contents/clostridium-difficile-in-adults-treatment, retrieved 14.03.2014).
*Clostridium difficile* infection recurs in about 20% of patients, and increases to 40% and 60% with subsequent recurrences (Kelly, C. P., LaMont, J. T., Clostridium difficile - more difficult than ever. N Engl J Med. 2008: 359, 1932-1940). Antimicrobial treatment disrupts the complex balance of diverse microorganisms and is a key factor in the pathogenesis of *C. difficile* colonization and disease of endogenous origin. Patients with recurrent *C. difficile* infection (CDI) have markedly diminished bacterial diversity compared with controls (Chang, J. Y., Antonopoulos, D. A., Kalra, A., Tonelli, A., Khalife, W. T., Schmidt, T. M., Young, V. B. Decreased diversity of the fecal Microbiome in recurrent C. difficile-associated diarrhea. J Infect Dis. 2008: 197, 435-438). Preservation and restoration of the microbial diversity could represent novel strategies. The crucial moment in prevention and treatment of this disease is to find the possibility to reconstitute the alteration of intestinal microbiota during and after antibiotic therapy with various regimens incl. administration of probiotics. Most probiotics colonize the gut temporarily, produce bactericidal acids and peptides and promote "competition" among microbes by competing for nutrients and epithelial adhesion. These effects appear to reduce the favorability of the environment for *C. difficile.* Previous studies suggest that probiotics for prevention of CDI include combination *L. acidophilus* and *L. casei, S. boulardii,* or *L. rhamnosus.* In addition, a dosage of >10⁹ CFU/day is more effective than lower doses.

The antimicrobial activity of probiotic strains is one of the suggested mechanisms for competition with *C. difficile.* Lactic acid bacteria produce short chain fatty acids that lower the pH of the local gut environment as well as prevent the adhesion of *C. difficile* (McFarland, L. V., Beneda, H. W., Clarridge, J. E., Raugi, G. J. Implications of the changing face of C. difficile disease for health care practitioners. Am J Infect Control. 2007: 35, 237-253). Next, the possibility for intestinal barrier protection with probiotics may result in interfering with the binding of *C. difficile* toxins A and B to colonic epithelial cells thus stabilizing gut permeability and inhibiting development of pseudomembranes on epithelia of gut. Studies have revealed that *Lactobacillus plantarum* strains are used for the prevention of cholesterol metabolism disorders, and that strains have anti-clostridial and LDL-lowering properties (De Vries M. C., Vaughan E. E., Kleerebezemac M., De Vos W. M. Lactobacillus plantarum-survival, functional and potential probiotic properties in the human intestinal tract, Int Dairy J, 2006 Vol 16, Issue 9, 1018-1028).
However, not all probiotics have the ability to produce mechanism of action described above.
Strain-specificity of lactobacilli is an important factor to take into consideration when looking for potential probiotics in the prevention of *C. difficile* infection or binding the *C. difficile* toxins (Tejero-Sariñena S., Barlow J., Costabile A, Gibson G. R., Rowland I. Antipathogenic activity of probiotics against Salmonella Typhimurium and Clostridium difficile in anaerobic batch culture systems: Is it due to synergies in probiotic mixtures or the specificity of single strains? Anaerobe. 2013: 24; 60-65).
Furthermore, some clinical trials could not reach statistical evidence to demonstrate the effect for the prevention of CDAD of certain probiotics. The authors of a large, randomized trial including 2941 elderly adults with antibiotic exposure noted that those who received probiotics (a multistrain preparation of *Lactobacillus acidophilus* and *Bifidobacterium bifidum*) did not show a risk reduction for CDI (RR 0.71; 95% CI 0.34-1.47; p =0.35) (Allen, S. J., Wareham, K., Wang, D., Bradley, C., Hutchings, H., Harris, W., Dhar, A., Brown, H., Foden, A., Gravenor, M. B., Mack, D. Lactobacilli and bifidobacteria in the prevention of antibiotic-associated diarrhoea and Clostridium difficile diarrhoea in older inpatients (PLACIDE): a randomised, double-blind, placebo-controlled, multicentre trial. Lancet. 2013: 382, 1249-1257). It is disclosed that *Lactobacillus plantarum* strains show high antagonistic activity against *Clostridium difficile* strains (Naaber, P., Smidt, I., Stsepetova, J., Brilene, T., Annuk, H., Mikelsaar, M., Inhibition of Clostridium difficile strains by intestinal Lactobacillus species. Journal of Medical Microbiology, 2004: 53 (6), 551-554). After years of trials with different probiotics for treatment of CDI the strain, dose, and duration of probiotics are still under discussion (Naaber, P., Mikelsaar, M., Interactions between Lactobacilli and antibiotic-associated diarrhea. Adv Appl Microbiol. 2004: 54 231-260).

### Xylitol application

Xylitol is a 5-C sugar alcohol, e.g. pentitol, and is found in plants, fungi and algae. Xylitol is an important intermediate product in mammalian carbohydrate metabolism; i.e. human blood contains up to 8x10⁻⁵ M of xylitol.
Consumed xylitol is not absorbed completely and the unabsorbed part can be used as a dietary fiber for bacterial fermentation to convert xylitol to short fatty acid chains utilized in energy pathways. Xylitol influences the growth of some species of gut microbiota in the large intestines stimulating the growth and activities of indigenous microbiota. One gram of xylitol contains 2.4 kcal as compared to one gram of glucose which has 3.87 kcal. Xylitol is advertised as "safe" for diabetics and individuals with hyperglycaemia (Talbot J. M., K. P. Fisher The Need for Special Foods and Sugar Substitutes by Individuals with Diabetes Mellitus. Diabetes Care 1978: 1; 231-240).
In our previous studies on CACO-2 cell lines we have discovered that 1% xylitol prevented the adhesion of vegetative cells of *C. difficile* reference strain VPI 10463, seemingly blocking the receptors on cells. In an applied hamster model (Naaber, P., Lehto, E., Salminen, S., Mikelsaar, M., Inhibition of adhesion of Clostridium difficile to Caco-2 cells. FEMS Immunol Med Microbiol. 1996: 14, 205-209) 1 ml of 20% xylitol solution together with *Lactobacillus rhamnosus* GG significantly protected animals from development of severe enterocolitis (Naaber, P., Lehto, E., Salminen, S., Mikelsaar, M., Inhibition of adhesion of Clostridium difficile to Caco-2 cells. FEMS Immunol Med Microbiol. 1996: 14, 205-209). In these experimental studies with xylitol in combination with probiotic *L. rhamnosus* GG the vegetative cells of *Clostridium difficile,* precultivated in laboratory anaerobic environment, have applied for inoculation of cell cultures or hamsters.
In opposite, in clinical practice or elderly home the infection develops from inoculation with *C. difficile* extremely resistant spores surviving in the aerobic environment of these facilities. The spores start to germinate inside the intestine of host.
Some authors have postulated that in the animal model some of sugars similarly to glucose could block the expression of toxins A and B of *C. difficile* (Karlsson, S., Burman, L. G., Akerlund, T. Induction of toxins in Clostridium difficile is associated with dramatic changes of its metabolism. Microbiology. 2008: 154, 3430-3436).

Thus there is still a need for probiotic strains effective in reducing LDL-cholesterol, especially the ox-LDL.

### DISCLOSURE OF THE INVENTION

The current invention provides the composition comprising *L. plantarum* Inducia strain DSM 21379, deposited in the Deutsche Sammlung von Mikroorganismen und Zellkulturen GmbH under the registration number DSM 21379 on 16.04.2008, for use as hypocholesterolemic agent in preventing the cholesterol metabolism disorders and consecutive cardiovascular disorders by reducing the level of LDL-cholesterol in blood, and simultaneously as an anti-oxidative agent for use in enhancing anti-oxidative activity of human body by reducing the levels of oxidative stress markers - oxidized low density lipoprotein (ox-LDL), oxidative stress index (OSI) and total peroxide count (TPX).

Account of this the compositions and food products comprising *L. plantarum* Inducia DSM 21379 have a cardio-protective effect.

The compositions comprising *L. plantarum* Inducia can be used for the production of food products, food supplements or pharmaceutical or veterinary products. The food products can be dairy or meat products, sweets, etc. Dairy products can be fermented milk products, cheese, and yoghurt, etc.
The food supplement may be used in powder (capsules, lozenges, tablets, powder sachets etc.) or liquid (ampoules) form. The strain may be also used in freeze-dried form.
The effective doze of *L. plantarum* Inducia strain DSM 21379 is about 1x10⁹ CFU to 1x10¹⁰CFU, preferably: 5x10⁹ CFU per day.
The term "comprising" herein encompasses "including" as well as "consisting", e.g. composition comprising *L. plantarum* Inducia may consist exclusively said strain or may include something additional.

### DESCRIPTION OF DRAWINGS

Fig. 1 The pH values of Inducia in xylitol containing and control modified MRS media in microaerobic and anaerobic environment.
Fig. 2 Presence of toxA and B genes in *C. difficile* VPI 10643 by reverse transcription (RT) and real-time PCR amplification (qPCR).
Fig. 3 Presence of toxB gene in liver and small intestine of a hamster treated with ampicillin and *C. difficile* by reverse transcription (RT) and real-time PCR amplification (qPCR).
Fig. 4 a) Normal mucosa of large intestine after probiotic administration and b) mucosa with *C. difficile* infection.
Fig. 5 Morphological changes in experimental CDAD infection. Moderate hyperemia in a) small intestine and b) large intestine and intense hyperaemia in c) pancreas and d) spleen. Pseudomembrane in e) small intestine and PMN infiltration with pseudomembrane f) in large intestine.

### DESCRIPTION OF THE EMBODIMENTS

The present invention is further illustrated in the following examples.

### I. Use of L. plantarum Inducia DSM 21379 as hypocholesterolemic agent by decreasing LDL-cholesterol in blood

### Example 1. Bile Salt Hydrolase activity of L. plantarum Inducia DSM 21379

The purpose of the following *in vitro* test was to evaluate the presence of bile salt hydrolase activity of *L. plantarum* Inducia DSM 21379.
Methods. Assessing the Bile Salt Hydrolase (BSH) activity *L. plantarum* Inducia DSM 21379 was performed according to Cuñé Castellana, 2009 (WO 2011/042333 A1, EP 2 485743 B1; AB Probiotics S.A.)
Three *Lactobacillus spp* strains were cultured overnight on MRS agar in microaerobic conditions at 37°C. After incubation, cultures were standardised to McFarland 3.0. The single-strain cultures were assayed for BSH activity. Cultures were impregnated around the sterilized paper disks on MRS agar plates supplemented with 4% (w/v) sodium salt of taurodeoxycholic acid (TDCA, Sigma, USA) and 0.37 g/l CaCl₂. Plates were anaerobically incubated at 37°C for 72 h, and the diameter of the precipitation zones around the disks was measured. BSH activity was then calculated by subtracting the disc diameter (DD, mm) from the inhibition zone diameter (IZD, mm) and dividing this difference by two following the formula BSH activity = (IZD-DD) / 2 (Table 1).

**Table 1. Bile salt hydrolase (BSH) activity of L. plantarum Inducia DSM 21379**

| | BSH activity (mm) |
|---|---|
| *L. plantarum* Inducia DSM 21379 | 1.7 |
| *L. plantarum* BAA 793 | 1.5 |
| *L. gasseri* DSM 23882 | 0.75 |

BSH activity of *L. plantarum* Inducia DSM 21379 was higher than reference *L. plantarum* strain BAA793 and *L. gasseri* DSM 23882 (Table 1).

### Example 2. LDL-cholesterol decreasing ability of L. plantarum Inducia DSM 21379

The purpose of the human intervention trial ISRCTN79645828 "Effect of probiotic yoghurt on blood indices and intestinal microflora of healthy volunteers" was to assess the short term (3 weeks) LDL-cholesterol decreasing ability of *L. plantarum* Inducia DSM 21379 in blood when consumed with yoghurt comprising said strain.

Yoghurt preparation. The probiotic yoghurt was developed from adjusted and pasteurized (+92...+95°C 5min) cow milk using *L. plantarum* Inducia DSM 21379 (2x10¹¹ CFU/g) as an adjunct starter (inoculation dose 1g/t milk). Shortly, the pasteurized milk was cooled to +35 ...+43°C before mixing with starter cultures and the probiotic strain. The milk was fermented until a pH 4.2 ... 4.5 was reached and cooled to +23...+27°C. The yoghurt was sweetened with 5% of sugar, packaged in plastic cups and cooled to +2...+6°C. A yoghurt without probiotic adjunct served as a control.

**Design of human volunteer trial.** The double-blind placebo-controlled (DBPC) cross-over exploratory trial was conducted according to the guidelines of Declaration of Helsinki. The trial was approved by the Ethics Review Committee on Human Research of the University of Tartu, Estonia (protocol number 190T-11, 2010). All participants signed their written informed consent at the enrolment and were given the possibility to withdraw from the study any time. The study was performed to investigate the effect of yoghurt comprising *L. plantarum* Inducia DSM 21379 on health biomarkers in healthy adults (n=49). Within one month prior to study participants were asked to continue their normal diet, and to avoid probiotic products (*e.g.* food supplements, yoghurts, cheese, kefir *etc*).
Two groups of participants started simultaneously with 3-week consumption of 150 g daily of test-yoghurt *i.e.* yoghurt comprising *L. plantarum* Inducia DSM 21379 (4x10⁷ CFU/g) or control yoghurt. The daily dose of the probiotic *L. plantarum* Inducia was 6 x 10⁹ CFU. After a two-week washout period, the volunteers were crossed over to another three weeks period of consumption of the probiotic yoghurt or control yoghurt.

### Clinical investigations

The subjects were clinically investigated and blood plasma samples were collected after an overnight fast and abstinence from any medications, tobacco, alcohol and tea or coffee. Each participant was evaluated for anthropometrical indices. Body mass index (BMI) was calculated as the weight (kg) divided by squared height (m²). BMI was used to classify normal weight range (18.5-24.9 kg/m²), overweight (≥25.0 kg/m²) and obesity (≥30.0 kg/m²) in healthy volunteers (WHO. The International Task Force. Obesity: Preventing and Managing the Global Epidemic. Report of a WHO Consultation on Obesity. Geneva, Switzerland. WHO/Nut/NCD/98. 1998; 1).
The samples of fasting blood were collected four times: at recruitment, after administration of either the *L. plantarum* Inducia DSM 21379 comprising product or control product, after wash-out period, and after the administration of the control or probiotic product at the end of the trial.

Haematological indices: plasma lipids: total cholesterol, LDL-cholesterol (LDL-c), HDL-cholesterol (HDL-c) and triglycerides were determined by standard laboratory methods using certified assays in the local clinical laboratory (United Laboratories of Tartu University Clinics, Estonia). Intervals for routine laboratory tests proposed by Nordic Reference Interval Project (NORIP, Rustard P., Felding P., Franszon L., Kairisto V., Lahti A., Martensson A., Hyltoft Petersen P., Simonsson P., Steensland H,. Uldall A. The Nordic Reference Interval Project 2000: recommended reference intervals for 25 common biochemical properties. Scand J Clin Lab Invest. 2004: 64: 271 - 284) were used as reference.

### Statistical analysis

Statistical analysis was performed by using R 2.10.1 (http://www.r-proiect.org Retrieved 25.03.2014) and GraphPad Prism version 4.00 for Windows (GraphPad Software, San Diego, CA). All data were expressed as mean and standard deviation (means ± SD). Baseline and intervention data were compared by paired t-test or Wilcoxon rank sum test according to the distribution of data.
Differences were considered statistically significant if the value was p < 0.05.

The two groups of healthy volunteers of the cross-over trial did not differ in their clinical data (Table 2).

**Table 2. Baseline values of healthy volunteers**

| | Group 1 n=25 | Group 2 n=24 | P value |
|---|---|---|---|
| Age | 35.8 ± 12.0 | 38.0 ± 12.7 | 0.617 |
| | 19.0 - 58.0 (34.0) | 19.0 - 62.0 (36.5) | |
| SexF/M)^{§} | 18/7 | 18/6 | 1.0 |
| HbAlc * | 5.5 ± 0.2 | 5.5 ± 0.3 | 0.976 |
| BMI | 23.8 ± 4.3 | 25.2 ± 5.2 | 0.297 |
| | 18.1 - 34.6 (22.7) | 18.6 - 43.4 (25.3) | |
| Waist//hip | 0.78 ± 0.06 | 0.79 ± 0.07 | 0.434 |
| | 0.68 - 0.97 (0.77) | 0.68 - 0.94 (0.79) | |
| Systolic blood pressure | 118.3 ± 10 | 121.6 ± 15.5 | 0.660 |
| | 101.0 -144.5 (118.0) | 98.5 - 158.0 (117.8) | |
| Diastolic blood pressure | 77.5 ± 7.4 | 78.6 ± 8.8 | 0.719 |
| | 63.5 - 92.5 (76.7) | 65.5 - 98.5 (79.3) | |
| Cholesterol | 5.1 ± 1.1 | 5.2 ± 0.9 | 0.952 |
| LDL-cholesterol | 3.4 ± 1.0 | 3.4 ± 1.2 | 0.873 |
| HDL-cholesterol | 1.7 ± 0.4 | 1.7 ± 0.5 | 0.719 |
| Triglycerides | 1.2 ± 1.0 | 1.1 ± 0.3 | 0.298 |
| Glucose | 4.9 ± 0.5 | 4.9 ± 0.4 | 0.560 |

| | | | |
|---|---|---|---|
| *glucohaemoglobin, ^{§} Fisher exact test | | | |

**Table 3. Metabolic indices of blood sera during the trial**

| | Probiotic yoghurt | | Control yoghurt | | *P* values *BL1 vs PRO / BL2 vs PL (BL1 vs BL2, PRO vs PL) |
|---|---|---|---|---|---|
| | Baseline1 | Probiotic period | Baseline 2 | Placebo period | |
| Cholesterol total | 5.2 ± 1.0 | 5.1 ± 0.9 | 5.1 ± 0.9 | 5.1 ± 0.9 | **0.081** / 1.0 |
| | 3.0 - 8.1 (5.1) | 3.2 - 7.1 (5.0) | 3.2 - 7.5 (4.9) | 3.5 - 7.7 (5.0) | (0.588, 0.172) |
| LDL-cholesterol total | 3.3 ± 1.0 | 3.0 ± 0.9 | 3.3 ± 1.1 | 3.1 ± 1.0 | **<0.001** / 0.099 |
| | 1.3 - 6.2 (3.3) | 1.2 - 5.0 (2.9) | 1.1 - 6.1 (3.2) | 0.9 - 5.6 (2.9) | (0.358, 0.329) |
| HDL-cholesterol total | 1.7 ± 0.5 | 1.7 ± 0.5 | 1.7 ± 0.5 | 1.7 ± 0.5 | 0.569 / 0.402 |
| | 0.8 - 3.1 (1.8) | 1.0 - 3.3 (1.7) | 0.9 - 3.0 (1.8) | 0.9 - 3.1 (1.7) | (0.896, 0.788) |
| Triglycerides total | 1.1 ± 0.8 | 1.1 ± 0.6 | 1.1 ± 0.6 | 1.1 ± 0.6 | 0.694 / 0.456 |
| | 0.4 - 4.4 (0.9) | 0.3 - 3.9 (0.9) | 0.5 - 4.0 (1.0) | 0.5 - 4.1 (1.0) | (0.834, 0.412) |

| | | | | | |
|---|---|---|---|---|---|
| *BL1: baseline 1; PRO: probiotic (i.e. *L. plantarum* Inducia comprising probiotic) periood; BL2: baseline 2; PL: placebo period. | | | | | |

The main changes after *L. plantarum* Inducia DSM 21379 consumption were revealed in the content of LDL-cholesterol particles. The LDL-cholesterol content was decreased after consumption of *L. plantarum* Inducia DSM 21379 comprising yoghurt (Table 3). Thus, the consumption *of L. plantarum* Inducia DSM 21379 yoghurt during 3 weeks reduced the LDL-cholesterol level for 9.1 % in blood.

### Example 3. Effect of L. plantarum Inducia DSM 21379 comprising yohgurt on oxidative stress markers oxidized low-density lipoprotein (oxLDL), oxidative stress index (OSI) and total peroxide count (TPX).

During the parallel-armed double-blind study ISRCTN26344255 mildly hypercholesterolemic (≥3.0 mmol/L for LDL-cholesterol) adults (n=136) consumed 150g daily the probiotic (daily dose of *L. plantarum* Inducia DSM 21379 being 5x10⁹ CFU) or control yoghurt for 8 weeks. The short term effect of the probiotic product was measured after 4-week of intervention and the sustained effect was measured at the end of the trial i.e. after 8 weeks of intervention.
The trial was approved by the Ethics Review Committee on Human Research of the University of Tartu, Estonia (protocol number 207/M-11 19.09.2011).

The consumption of 150g yoghurt comprising *L. plantarum* Inducia DSM 21379 in an average daily dose of 5x10⁹ CFU resulted in a steady intra-group reduction in LDL-cholesterol in probiotic group between week 4 and 8 week (-0.12mmol/L; p=0.04) and from baseline to week 8 (-0.09 mmol/L; p=0.029) (Table 4a and 4b).

Ox-LDL is a marker of oxidative stress specific to LDL particles, and therefore ox-LDL could be considered one of biochemical risk markers for coronary heart diseases. The administration of yoghurt comprising *L. plantarum* Inducia DSM 21379 resulted in steady intra group reduction of ox-LDL in probiotic yoghurt group in throughout 8-week intervention (Table 5a and 5b). The change was statistically significant from baseline to week 8 (-4.10U/L; p=0.003). The change was also significant over the placebo (p=0.026). No effect on oxLDL was registered in placebo group.

The administration of probiotic yoghurt comprising *L. plantarum* Inducia DSM 21379 resulted also in significant (-5.37; p<0.001) reduction of oxidative stress index (OSI) from baseline to week 8 in probiotic group (Table 6a and 6b). The change was also significant over the placebo (p=0.04). No effect was registered in placebo group.

**Table 4a. LDL-cholesterol (mmol/L) levels during visits**

| | Treatment | | | |
|---|---|---|---|---|
| | | *L. plantarum* Inducia | Placebo | p-value^{a} |
| W0: LDL | Mean | 3.56 | 3.6 | 0.804 |
| | Std | 0.59 | 0.67 | |
| | Min | 2.52 | 2.42 | |
| | Q1 | 3.12 | 3.08 | |
| | Median | 3.5 | 3.52 | |
| | Q3 | 3.86 | 4.06 | |
| | *Max* | 5.06 | 5.27 | |
| W4: LDL | Mean | 3.56 | 3.59 | 0.552 |
| | Std | 0.64 | 0.73 | |
| | Min | 2.66 | 2.19 | |
| | Q1 | 3.08 | 3.02 | |
| | Median | 3.49 | 3.66 | |
| | Q3 | 3.93 | 4.09 | |
| | Max | 5.38 | 4.97 | |
| W8: LDL | Mean | 3.48 | 3.59 | 0.350 |
| | Std | 0.67 | 0.69 | |
| | Min | 2.13 | 2.29 | |
| | Q1 | 2.96 | 3.04 | |
| | Median | 3.38 | 3.59 | |
| | Q3 | 3.9 | 4.06 | |
| | Max | 5.32 | 5.35 | |

| | | | | |
|---|---|---|---|---|
| ^{a}Wilcoxon Rank Sum test; W0: visit 2, start of the intervention; W4: visit 3, after 4 week of intervention;W8: visit 4, after 8 week of intervention. | | | | |

**Table 4b. Change in LDL-cholesterol (mmol/L) levels during 8-week administration of yoghurt comprising L. plantarum Inducia DSM 21379**

| | Treatment | | | |
|---|---|---|---|---|
| | | *L.plantarun* Inducia | Placebo | p-value |
| | Mean | 0.01 | -0.02 | 0.629 |
| W4 difference from W0 | Std | 0.41 | 0,48 | |
| | Min | -0.85 | -0.93 | |
| LDL | Q1 | -0.28 | -0.33 | |
| | Median | -0.01 | -0.07 | |
| | Q3 | 0.22 | 0.22 | |
| | Max | 1.35 | 1.42 | |
| | p-value^{b} | 0,700 | 0,443 | |
| | Mean | -0.09 | -0.01 | 0.207 |
| | Std | 0.43 | 0.4 | |
| W8 difference from W0 | Min | -1.01 | -0.83 | |
| | Q1 | -0.39 | -0.2 | |
| LDL | Median | -0.13 | -0.02 | |
| | Q3 | 0.15 | 0.17 | |
| | Max | 1.41 | 1.4 | |
| | p-value^{b} | 0.029 | 0.847 | |
| | Mean | -0.12 | 0.03 | 0.058 |
| | Std | 0.42 | 0.43 | |
| W8 difference from W4 | Min | -1.68 | -1.17 | |
| | Q1 | -0.31 | -0.3 | |
| LDL | Median | -0.08 | 0.07 | |
| | Q3 | 0.11 | 0.28 | |
| | Max | 0.79 | 0.83 | |
| | p-value^{b} | 0.040 | 0.357 | |

| | | | | |
|---|---|---|---|---|
| ^{a}Wilcoxon Rank Sum test; ^{b}Wilcoxon Signed Rank test; W0: visit 2, start of the intervention; W4: visit 3, after 4 week of intervention; W8: visit 4, after 8 week of intervention, p-value^{a}: inter-group (between verum and placebo) significance p-value^{b} intra-group significance. | | | | |

**Table 5a. Ox-LDL-cholesterol (mmol/L) levels during visits**

| | Treatment | | | |
|---|---|---|---|---|
| | | *L. plantarum* Inducia | Placebo | p-value^{a} |
| | Mean | 67.95 | 61.41 | 0.068 |
| *W0*: | Std | 17.75 | 17.95 | |
| *ox-LDL* | Min | 37.6 | 32,7 | |
| | Q1 | 53.95 | 46.4 | |
| | Median | 66.2 | 55.6 | |
| | Q3 | 83 | 74 | |
| | Max | 108.3 | 108.2 | |
| | Mean | 66.31 | 62.02 | 0.234 |
| | Std | 17.27 | 17.87 | |
| *W4:* | Min | 40.7 | 34.4 | |
| *ox-LDL* | Q1 | 51.9 | 47.9 | |
| | Median | 61.5 | 56.7 | |
| | Q3 | 77.1 | 76.6 | |
| | Max | 111.2 | 100 | |
| | Mean | 62.9 | 61.58 | 0.575 |
| | Std | 15.48 | 16.97 | |
| *W8*: | Min | 40.1 | 37.4 | |
| *ox-LDL* | Q1 | 48.6 | 47.1 | |
| | Median | 62.05 | 61.7 | |
| | Q3 | 73.15 | 74.2 | |
| | Max | 99.2 | 105.6 | |

| | | | | |
|---|---|---|---|---|
| ^{a}Wilcoxon Rank Sum test; W0: visit 2, start of the intervention; W4: visit 3, after 4 week of intervention; W8: visit 4, after 8 week of intervention. | | | | |

**Table 5b. Change in LDL-cholesterol (mmol/L) levels during 8-week administration of yoghurt comprising L. plantarum Inducia DSM 21379**

| | Treatment | | | |
|---|---|---|---|---|
| | | *L.plantarun* Inducia | Placebo | p-value^{a} |
| W4 difference from W0 ox-LDL | Mean | -1.85 | 0.61 | 0.116 |
| | Std | 7.27 | 9.2 | |
| | Min | -17.7 | -15.7 | |
| | Q1 | -6.7 | -3.9 | |
| | Median | -1.6 | 0.2 | |
| | Q3 | 2 | 4.7 | |
| | Max | 20.2 | 37.9 | |
| | p-value^{b} | 0.055 | 0.811 | |
| | Mean | -4.1 | 0.55 | 0.026 |
| W8 difference from W0 | Std | 8.84 | 10.57 | |
| | Min | -26 | -27.4 | |
| ox-LDL | Q1 | -8.1 | -5.3 | |
| | Median | -3.95 | 0.2 | |
| | Q3 | 1.05 | 6.3 | |
| | Max | 14 | 32.3 | |
| | p-value^{b} | 0.003 | 0.726 | |
| W8 difference from W4 | Mean | -2.78 | -0.16 | 0.097 |
| | Std | 8.6 | 9.41 | |
| ox-LDL | Min | -24.4 | -31.6 | |
| | Q1 | -8.3 | -6.1 | |
| | Median | -2.9 | 1.4 | |
| | Q3 | 3 | 4.6 | |
| | Max | 16.2 | 16.6 | |
| | *p*-value^{b} | 0.039 | 0.652 | |

| | | | | |
|---|---|---|---|---|
| ^{a}Wilcoxon Rank Sum test; ^{b}Wilcoxon Signed Rank test; W0: visit 2, start of the intervention; W4: visit 3, after 4 week of intervention; W8: visit 4, after 8 week of intervention; p-value^{a}: inter-group (between verum and placebo) significance p-value^{b}: intra-group significance. | | | | |

**Table 6a. Oxidative stress index (OSI) during visits**

| | Treatment | | | |
|---|---|---|---|---|
| | | *L.plantarun* Inducia | Placebo | *p-value^{a}* |
| | Mean | 29.11 | 34.29 | 0.230 |
| W0: OSI | Std | 17.92 | 19.98 | |
| | Min | 5.36 | 0 | |
| | Q1 | 14.7 | 19.24 | |
| | Median | 24.84 | 31.8 | |
| | Q3 | 40.52 | 48.03 | |
| | Max | 71.05 | 94.71 | |
| | Mean | 27.27 | 32.17 | 0.210 |
| W4: OSI | Std | 18.85 | 19.8 | |
| | Min | 4.83 | 0 | |
| | Q1 | 12.14 | 16.25 | |
| | Median | 25.09 | 29,07 | |
| | Q3 | 35.47 | 46.78 | |
| | Max | 72.59 | 75.98 | |
| | Mean | 24.13 | 33.24 | 0.011 |
| W8: OSI | Std | 15.31 | 17.98 | |
| | Min | 4 | 0 | |
| | Q1 | 13.65 | 17.59 | |
| | Median | 20.62 | 35.03 | |
| | Q3 | 31.87 | 46.61 | |
| | Max | 61.49 | 65.77 | |

| | | | | |
|---|---|---|---|---|
| ^{a}Wilcoxon Rank Sum test; W0: visit 2, start of the intervention; W4: visit 3, after 4 week of intervention; W8: visit 4, after 8 week of intervention. | | | | |

**Table 6b. Change in oxidative stress index (OSI) during 8-week administration of yoghurt comprising L. plantarum Inducia DSM 21379**

| | Treatment | | | |
|---|---|---|---|---|
| | | *L. plantarum* Inducia | Placebc | p-value^{a} |
| | Mean | -0.8 | -1.3 | 0.848 |
| | Std | 7.82 | 10.27 | |
| W4 difference from W0 | Min | -22.3 | -22.61 | |
| OSI | Q1 | -4.01 | -7.25 | |
| | Median | -0.46 | 1.09 | |
| | Q3 | 3.23 | 4.05 | |
| | Max | 30.08 | 27.2 | |
| | p-value^{b} | 0.505 | 0.696 | |
| | Mean | -5.37 | -1.69 | 0.040 |
| W8 difference from W0 | Std | 8.47 | 11.41 | |
| | Min | -32.84 | -35 | |
| OSI | Q1 | -8.72 | -7.85 | |
| | Median | -3.95 | 0 | |
| | Q3 | -0.42 | 5.25 | |
| | Max | 16.99 | 25.09 | |
| | p-value^{b} | <0.001 | 0.568 | |
| | Mean | -4.73 | 1.1 | 0.010 |
| W8 difference from W4 | Std | 7.04 | 14.77 | |
| | Min | -22.55 | -32.18 | |
| OSI | Q1 | -8.55 | -7.03 | |
| | Median | -3.82 | 1.39 | |
| | Q3 | -0.11 | 6.87 | |
| | Max | 11.78 | 60.39 | |
| | p-value^{b} | <0.001 | 0.717 | |

| | | | | |
|---|---|---|---|---|
| ^{a}Wilcoxon Rank Sum test; ^{b}Wilcoxon Signed Rank test; W0: visit 2, start of the intervention; W4: visit 3, after 4 week of intervention; W8: visit 4, after 8 week of intervention; p-value^{a}: inter-group (between verum and placebo) significance p-value^{b}: intra-group significance. | | | | |

**Table 7a. Total peroxide count (TPX, mol/L) during visits**

| | Treatment | | | |
|---|---|---|---|---|
| | | *L. plantarum* Inducia | Placebo | p-value^{a} |
| W0: TPX | Mean | 443.98 | 516.53 | 0.247 |
| | Std | 255.3 | 289.82 | |
| | Min | 81 | 75 | |
| | Q1 | 255 | 300 | |
| | Median | 397.5 | 524.5 | |
| | Q3 | 644 | 690 | |
| | Max | 945 | 1468 | |
| W4: TPX | N | 46 | 38 | 0.189 |
| | Mean | 414.8 | 498.05 | |
| | Std | 257.56 | 288.77 | |
| | Min | 73 | 102 | |
| | Q1 | 175 | 250 | |
| | Median | 363.5 | 422.5 | |
| | Q3 | 573 | 700 | |
| | Max | 924 | 1284 | |
| W8: TPX | N | 45 | 37 | 0.009 |
| | Mean | 367.84 | 502.92 | |
| | Std | 209.55 | 240.8 | |
| | Min | 64 | 77 | |
| | Q1 | 210 | 316 | |
| | Median | 346 | 465 | |
| | Q3 | 469 | 737 | |
| | Max | 838 | 1015 | |

| | | | | |
|---|---|---|---|---|
| ^{a}Wilcoxon Rank Sum test; W0: visit 2, start of the intervention; W4: visit 3, after 4 week of intervention; W8: visit 4, after 8 week of intervention. | | | | |

Steady significant intergroup reduction of total peroxide count (TPX) was registered in probiotic (i.e. *L. plantarum* Inducia comprising probiotic) group throughout the study (Table 7a and 7b). No effect was registered in placebo group. No significant change was detected in total antioxidative capacity (Table 8a and 8b).

**Table 7b. Change in total peroxide count (TPX, mol/L) during 8-week administration of yoghurt comprising L. plantarum Inducia DSM 21379**

| | Treatment | | | |
|---|---|---|---|---|
| | | *L. plantarum* Inducia | Placebo | p-value^{a} |
| | Mean | -23.48 | -18.47 | 0.903 |
| W4 difference from W0 | Std | 77.07 | 137.07 | |
| | Min | -271 | -325 | |
| TPX | Q1 | -51 | -72 | |
| | Median | -15 | -18.5 | |
| | Q3 | 21 | 62 | |
| | Max | 143 | 383 | |
| | p-value^{b} | 0.044 | 0.416 | |
| | Mean | -84.22 | -36 | 0.227 |
| W8 difference from WO | Std | 116.4 | 147.78 | |
| | Min | -473 | -453 | |
| TPX | Q1 | -115 | -118.5 | |
| | Median | -56 | -54.5 | |
| | Q3 | -26 | 68.5 | |
| | Max | 84 | 261 | |
| | p-value^{b} | <0.001 | 0.225 | |
| | Mean | -67.91 | -17.61 | 0.052 |
| W8 difference from W4 | Std | 113.93 | 155.41 | |
| | Min | -486 | -466 | |
| TPX | Q1 | -87 | -89.5 | |
| | Median | -55 | -3 | |
| | Q3 | -2 | 67 | |
| | Max | 114 | 295 | |
| | p-value^{b} | <0.001 | 0.758 | |

| | | | | |
|---|---|---|---|---|
| ^{a}Wilcoxon Rank Sum test; ^{b}Wilcoxon Signed Rank test; W0: visit 2, start of the intervention; W4: visit 3, after 4 week of intervention; W8: visit 4, after 8 week of intervention; p-value^{a} : inter-group (between verum and placebo) significance p-value^{b}: intra-group significance. | | | | |

**Table 8a. Total antioxidant capacity (TAC, mmol Trolox equivalent/L) levels during visits**

| | Treatment | | | |
|---|---|---|---|---|
| | | *L. plantarum* Inducia | Placebo | p-value^{a} |
| W0: TAC | Mean | 1.52 | 1.48 | 0.361 |
| | Std | 0.25 | 0.19 | |
| | Min | 1.01 | 1.03 | |
| | Q1 | 1.34 | 1.34 | |
| | Median | 1.52 | 1.44 | |
| | Q3 | 1.69 | 1.58 | |
| | Max | 2.01 | 1.94 | |
| W4: TAC | Mean | 1.49 | 1.47 | 0.541 |
| | Std | 0.23 | 0.19 | |
| | Min | 0.93 | 1.13 | |
| | Q1 | 1.33 | 1.35 | |
| | Median | 1.53 | 1.43 | |
| | Q3 | 1.69 | 1.63 | |
| | Max | 1.84 | 1.85 | |
| W8: TAC | Mean | 1.53 | 1.46 | 0.106 |
| | Std | 0.21 | 0.2 | |
| | Min | 1.12 | 1.12 | |
| | Q1 | 1.43 | 1.29 | |
| | Median | 1.53 | 1.46 | |
| | Q3 | 1.61 | 1.66 | |
| | Max | 2,.07 | 1.9 | |

| | | | | |
|---|---|---|---|---|
| ^{a}Wilcoxon Rank Sum test; W0: visit 2, start of the intervention; W4: visit 3, after 4 week of intervention; W8: visit 4, after 8 week of intervention. | | | | |

**Table 8b. Change in total antioxidant capacity (TAC, mmol Trolox equivalent/L) levels during 8-week administration of yoghurt comprising L. plantarum Inducia DSM 21379**

| | Treatment | | | |
|---|---|---|---|---|
| | | *L. plantarum* Inducia | Placebo | p-value^{a} |
| | Mean | -0.03 | -0.01 | 0.256 |
| W4 difference from W0 | Std | 0.23 | 0.15 | |
| | Min | -0.51 | -0.44 | |
| TAC | Q1 | -0.14 | -0.06 | |
| | Median | -0.05 | 0.01 | |
| | Q3 | 0.09 | 0.1 | |
| | Max | 0.63 | 0.23 | |
| | p-value^{b} | 0.274 | 0.692 | |
| | Mean | 0 | -0.02 | 0.891 |
| W8 difference from W0 | Std | 0.22 | 0.19 | |
| | Min | -0.49 | -0.44 | |
| TAC | Q1 | -0.13 | -0.13 | |
| | Median | 0.01 | -0.03 | |
| | Q3 | 0.1 | 0.14 | |
| | Max | 0.52 | 0.44 | |
| | p-value^{b} | 0.811 | 0.574 | |
| | Mean | 0.04 | -0.01 | 0.237 |
| W8 difference from W4 | Std | 0.2 | 0.16 | |
| | Min | -0.41 | -0.36 | |
| TAC | Q1 | -0.11 | -0.12 | |
| | Median | 0.06 | 0.02 | |
| | Q3 | 0.13 | 0.08 | |
| | Max | 0.66 | 0.38 | |
| | p-value^{b} | 0.342 | 0.706 | |

| | | | | |
|---|---|---|---|---|
| ^{a}Wilcoxon Rank Sum test; ^{b}Wilcoxon Signed Rank test; W0: visit 2, start of the intervention; W4: visit 3, after 4 week of intervention; W8: visit 4, after 8 week of intervention; p-value^{a}: inter-group (between verum and placebo) significance; p-value^{b}: intra-group significance. | | | | |

**Conclusion.** According to the human intervention trials the composition comprising *L*. *plantarum* Inducia DSM 21379 has cardio-protective effect through reduction of LDL-cholesterol in blood and simultaneously protects human body from oxidative damage and enhances anti-oxidative activity by reducing oxidative stress markers oxLDL, OSI and TPX.

### II. Comparative Examples, no part of the invention. Use of L. plantarum Inducia strain DSM 21379 as antimicrobial agent by preventing germination of spores of C.difficile and prolifertation of vegetative cells.

### Example 4. Antagonistic activity of L. plantarum Inducia DSM 21379 against vegetative cells of C difficile reference strains in anaerobic environment in vitro

The purpose of the study was to assess the antagonistic activity of *L. plantarum* Inducia DSM 21379 against *C difficile* reference strains in anaerobic environment.

Methods. The tested strains were: *L. plantarum* Inducia DSM 21379 and *C. difficile* VPI 10463 (ATCC 43255), M 13042 hypervirulent strain.
Strains were seeded on solid media and incubated in an anaerobic environment. The suspension of bacteria in physiological saline was adjusted according to MacFarland standard 3 (for 10⁸ CFU/ml). Suspension (0.2 ml) in the 200 ml BHI media was inoculated for reaching the concentration 10⁵ CFU/ml.
On different timescale the bacteriological seedings for growth were performed (0.1 ml) to MRS and LAB160 media. The plates were incubated in anaerobic and microaerobic conditions. The growth was checked after 2-5 days and the results were expressed as log₁₀ CFU/ml.

**Table 9. Suppression of proliferation of C. difficile reference strains after co-cultivation with L. plantarum Inducia DSM 21379 in BHI media after 0, 24, 48 h**

| Proliferation of strains as single culture in BHI | Growth (log CFU/ml) | | |
|---|---|---|---|
| | 0 h | 24 h | 48h |
| *L. plantarum* Inducia | 5.3 | 8.0 | 6.3 |
| | 5.8 | 4.6 | 7.5 |
| M 13042 clinical reference strain | 5.3 | 5.3 | 7.5 |
| Proliferation of *L. plantarum* Inducia and *C. difficile* strains after co-cultivation in BHI | 0 h | 24 h | 48 h |
| *L. plantarum* Inducia with *C*. *difficile* VPI | 5.8 | 8.8 | 7.3 |
| *L. plantarum* Inducia with M reference strain | 5.6 | 8.5 | 6.0 |
| *C. difficile* VPI 10463 strain with *L. plantarum* Inducia | 5.5 | 0 | 0 |
| *C. difficile* M 13042 strain with *L. plantarum* Inducia | 5.3 | 0 | 0 |

Complete suppression of *C. difficile* vegetative cells by *L. plantarum* Inducia DSM 21379 was discovered after co-cultivation in BHI medium further seeded on selective MRS and CD LAB180 media after 48 h incubation *L. plantarum* Inducia DSM 21379 showed the highest values at 24 h in the BHI medium. After 48 h the growth yield of *L*. *plantarum* Inducia DSM 21379 was modestly suppressed equally in single culture (6.3 log CFU/g) or if combined with *C. difficile* strains (7.3 and 6.0 log CFU/g)(Table 9).

### Example 5. Effect of supernatant of Lactobacillus plantarum Inducia DSM 21379 on vegetative cells of Clostridium difficile reference and clinical strains in vitro

The purpose of the following *in vitro* experiment was to determine the antimicrobial effect of *L. plantarum* Inducia DSM 21379 vegetative cells of on reference and clinical *C. difficile* strains. The distinction between the suppressive effect of natural (acidic) and neutralised supernatant (inhibitory substances e.g. peptides) of *L. plantarum* Inducia DSM 21379 helps to discriminate between two mechanisms - either impact of organic acids or presence of bacteriocins.
Material (strains) (Table 10)
*L. plantarum* Inducia DSM 21379
*Clostridium difficile* reference strains VPI 10463 and M 13042
*Clostridium difficile* clinical strains (11 strains)

Methods. Antimicrobial activity of *L. plantarum* Inducia DSM 21379 supernatant was determined against reference and clinical *C. difficile* strains by a microtitre plate (MTP) assay.

The *L. plantarum* Inducia DSM 21379 was maintained at -80°C in microtubes on glass-beads and was activated trice in MRS broth with 0.15% agar, incubated under microaerobic conditions (10% CO₂) at 37°C for 24 h. Overnight *L. plantarum* Inducia DSM 21379 culture was used to inoculate BHI broth 1% v/v and was incubated in microaerobic conditions for 24h. For detection of antimicrobial activity of *L. plantarum* Inducia DSM 21379, the extracellular cell free supernatant (CFS) was collected by centrifugation from a 24 h old BHI broth cultures. The pH of cell free supernatant was measured and divided in half. The on half was left acidic and the other half was neutralized with 6N NaOH to pH 6.0, the both supernatants were filter sterilized.

*C. difficile* strains were maintained at -80°C in microtubes on glass-beads and were activated trice on Fastidious Anaerobe Agar (FAA) with horse blood supplement for 24h in anaerobic milieu (Anaerobic glove box, gases 90%N:5%CO₂:5% H₂). Overnight *C. difficile* cultures were used for the suspension with density according to MacFarland 3.0. For evaluating the antimicrobial activity of *L. plantarum* Inducia DSM 21379, 20 µl of *C. difficile* cell suspension was added to 180 µl: (1) BHI broth (as control), (2) cell free BHI supernatant, (3) cell free neutralized BHI supernatant, (4) diluted cell free supernatant (1:1 in sterile BHI broth) and (5) diluted neutralized cell free supernatant (1:1 in sterile BHI broth).

Growth of *C. difficile* (change in optical density values) was measured after 48h at OD₆₂₀ₙₘ using an MTP reader and the growth rates were calculated.

The suppressive activity of *L. plantarum* Inducia DSM 21379 was tested with Kruskal-Wallis test, where the growth density (OD₆₂₀ₙₘ) of *C. difficile* control was compared with the data of *C. difficile* growth density in natural and neutralised and in supernatants dilutions. The statistical analysis of data was performed using PAST Statistics Web provided program.

**Table 10. Antimicrobial activity L. plantarum Inducia DSM 21379 supernatant neutral (acidic) and neutralised cell free supernatant and supernatant dilution against clinical and reference strains of C. difficile after 48h of cultivation.**

| *C. difficile* strains | The density of *C. difficile* in BHI (control) | The density of *C. difficile* in *L. plantarum* Inducia supernatant (natural or neutralized) | | The density of *C. difficile* in *L. plantarum* Inducia supernatant dilution 1:1 (supernatant: BHI) (natural or neutralized) | |
|---|---|---|---|---|---|
| | | natural | neutralized | natural | neutralized |
| CDE | 0.259 | 0.023 | 0.150 | 0.113 | 0.395 |
| CDP1 | 0.478 | 0.007 | 0.305 | -0.007 | 0.294 |
| CDP2 | 0.510 | 0.001 | 0.550 | 0.193 | 0.651 |
| CDP3 | 0.493 | 0.009 | 0.363 | 0.114 | 0.334 |
| CDP4 | 0.386 | 0.004 | 0.247 | 0.084 | 0.343 |
| CDP5 | 0.311 | 0.003 | 0.192 | 0.097 | 0.369 |
| CDP6 | 0.503 | 0.003 | 0.208 | 0.107 | 0.264 |
| CDP7 | 0.402 | 0.091 | 0.315 | 0.126 | 0.325 |
| CDP8 | 0.110 | 0.002 | 0.170 | 0.019 | 0.316 |
| CDP9 | 0.090 | 0.032 | 0.170 | 0.103 | 0.334 |
| CDP10 | 0.209 | 0.023 | 0.050 | 0.093 | 0.331 |
| VPI 10643 | 0.420 | 0.044 | 0.076 | 0.329 | 0.290 |
| M 13042 | 0.402 | 0.005 | 0.249 | 0.083 | 0.304 |
| Mean ± SD | 0.352±0.15^{a;b ;c} | 0.019±0.0 3^{a;d;e;f} | 0.234±0.13^{b ;d} | 0.112±0.08^{c} ;^{e} | 0.350±0.10^{f} |

| | | | | | |
|---|---|---|---|---|---|
| ^{a}p<0.0001; ^{b}p<0.05; ^{c}p<0.01; ^{d}p<0.001; ^{e}p<0.01; ^{f}p<0.001 | | | | | |

Vegetative cells of clinical strains: *C. difficile* CDE, CDP 1-9; reference strains: C. difficile VPI 10463 (ATCC 43255) and *C. difficile* M13042 (epidemic strain from Canada belonging to ribotype 027).

Thus, the strain *L. plantarum* Inducia DSM 21379 possess antimicrobial activity against *C. difficile* relying both on acid production in natural product (pH lowering) and in a smaller extent also on some antimicrobial protein-like substance still active after neutralisation of the supernatant (neutralized product) (Table 10).

### Example 6. Testing of growth and pH values of L. plantarum Inducia DSM 21379 in the media with xylitol

The purpose of the study was to find if xylitol influences the antagonistic activity of *L. plantarum* Inducia DSM 21379 against *C. difficile.*

Inducia (10⁵ CFU/ml) was incubated in MRS media where glucose was substituted for 5% xylitol or used without sugar in microaerobic and anaerobic environment. The count of lactobacilli was registered as CFU/ml of media. We tested at 2, 6, 24, and 48h if *L. plantarum* Inducia DSM 21379 uses xylitol for growth *in vitro* in microaerobic and anaerobic conditions.

At 24 h there was no difference in growth of *L. plantarum* Inducia DSM 21379 using media provided with xylitol or without it, at 24 h the growth in 5% of xylitole media was the best in microaerobic environment but a 2 log lower in anaerobic environment than that with glucose (Table 11).

**Table 11. Counts of L. plantarum Inducia DSM 21379 (log CFU/ml) after growth in anaerobic and microaerobic conditions in modified MRS medium with 5% of xylitol or glucose**

| Growth conditions | Sugar in the medium | The count of *L. plantarum* (log CFU/ml) after different time of incubation | | | | |
|---|---|---|---|---|---|---|
| | | 0 | 2h | 6 h | 24 h | 48 h |
| Microaerobic | xylitol | 5.6 | 5.60 | 6.24 | 8.0 | 7.2 |
| | glucose | 5.41 | 5.48 | 6.94 | 8.8 | 9.0 |
| Anaerobic | xylitol | 5.6 | 5.70 | 6.00 | 7.4 | 7.15 |
| | glucose | 5.41 | 5.70 | 6.96 | 9.1 | 9.1 |

This experiment proved that *L. plantarum* Inducia DSM 21379 did not effectively metabolise xylitol in MRS media. The change of pH after growth of *L. plantarum* Inducia DSM 21379 in 5% xylitol containing modified MRS media was tested.

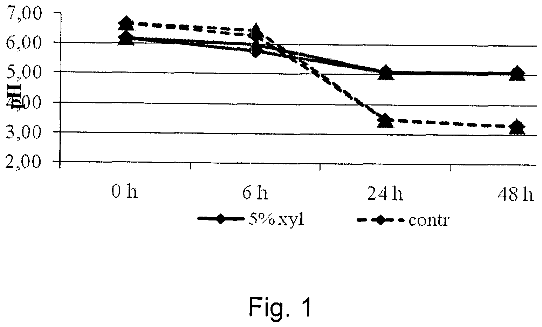

The lowest pH values by production of organic acids were seen in control media with glucose incubated in anaerobic environment. In xylitol containing media still a pH drop from pH 6.2 to pH 5.0 was seen both in microaerobic and anaerobic conditions, still substantially different from pH of control media with glucose from pH 7.5 to pH=3.2 (Fig 1).

### Use of xylitol in metabolism of L. plantarum Inducia DSM 21379

The purpose of the study was to measure the use of xylitol by *L. plantarum* Inducia DSM 21379 in microaerobic and anaerobic environments.

*Methods:* Double experiments were performed by cultivation of *L. plantarum* Inducia DSM 21379 in in MRS medium with 5% xylitol in microaerobic and anaerobic environments for 2 to 120 h. Xylitol was detected with mass spectrometry QTRAP 3200 (Applied Biosystems, USA). The samples were centrifuged 3 min 10000 g, diluted 100 folds and 50 µl was mixed with 50 µl internal standard (5 mM D4-succinic acid in acetonitrile, 50 µl). 5 µl of dilutions were injected into mass spectrometry using 50% acetonitril/water eluent. The substrates were identified by multiple reaction monitoring (MRM) ionpairs 151/101 (xylitol) and 121/77 (internal standard). Concentration was calculated from a calibration curve made from solutions with known concentrations of commercial xylitol (Sigma-Aldrich, Germany).

In the repeated experiments Inducia used xylitol in very low amounts as the content of the sugar was sustained at Basal value ± up to 0 - 0.6 mM of change (Table 12).

**Table 12. Xylitol content (mM) after cultivation of L. plantarum Inducia DSM 21379 in 5% xylitol media microaerobically and anaerobically during 120 h**

| Xylitol 1 (mM) | Repeated tests | 0 h | 2 h | 6 h | 24 h | 48 h | 120 h |
|---|---|---|---|---|---|---|---|
| 5% xylitol micro aerobic | I | 3.1 | ND | 3.2 | 2.5 | 3.2 | ND |
| | II | 3.4 | 3.4 | 3.3 | 3.2 | 3.3 | 3.3 |
| 5% xylitol anaerobic | I | 3.1 | ND | 2.8 | 3.2 | 2.5 | ND |
| | II | 3.4 | 3.4 | 3.2 | 3.2 | 3.3 | 3.5 |

| | | | | | | | |
|---|---|---|---|---|---|---|---|
| ND -not determined | | | | | | | |

Thus, we confirmed by mass spectrometry that *L. plantarum* Inducia metabolises xylitol in very low amounts.

### Growth of C difficile reference strains in environment containing xylitol and ampicillin

The purpose of the study was to mimic *in vitro* the gut environment similar to elaborated *C. difficile* infection model in Syrian hamsters.

*In vitro* the impact of xylitol and ampicillin on growth of *L. plantarum* Inducia DSM 21379 and reference strains of *C. difficile* (VPI 10463 and the hypertoxic Ribotype 027 strain M13042) in Brain Heart medium (BHI) was tested.

**Table 13. Impact of different concentrations of xylitol (0.1-5%) and ampicillin (0.75 µl/ml) on 48 h growth of Clostridium difficile and L. plantarum Inducia DSM 21379 as single in anaerobic milieu and BHI medium**

| Experimental modulators | *L. plantarum* Inducia log cfu/g | | *C. difficile* VPI log cfu/g | | *C.difficile* M strain log cfu/g | |
|---|---|---|---|---|---|---|
| | 0 h | 48 h | 0 h | 48 | 0 h | 48 h |
| BHI control | 5.9 | 9.8 | 5.3 | 7.2 | 4.9 | 7.1 |
| 0.1 % xylitol | 5.3 | 8.0 | 4.7 | 6.4 | 5.0 | 6.9 |
| 1 % xylitol | 5.0 | 8.0 | 5.5 | 7.3 | 4.8 | 6.7 |
| 2.5 % xylitol | 5.5 | 8.3 | 5.0 | 6.7 | 5.0 | 6.0 |
| 5 % xylitol | 5.5 | 7.8 | 5.0 | 7.1 | 5.0 | 6.7 |
| Ampicillin 0.75 µl/ml | 5.3 | 7.1 | 5.8 | 7.0 | 4.7 | 7.0 |

The *L. plantarum* Inducia DSM 21379 and the two reference strains of *C. difficile* increased the number of CFU nearly for 2 to 4 logarithms after 48 h of cultivation in the control media of BHI. No changes were seen after influencing *C. difficile* strains with xylitol and ampicillin (Table 13).

Growth of strain *L. plantarum* Inducia DSM 21379 was suppressed for ∼2 logarithms both under 5% of xylitol and 0.75 µl/ml of ampicillin. Thus, in gut microenvironment with administered antibiotic ampicillin and up to 5% xylitole the survival of *L.plantarum* Inducia is granted.

### Example 7. Growth of L. plantarum Inducia DSM 21379 and C.difficile reference strains by co-cultivation at different concentrations of xylitol and ampicillin

The purpose of the study was to assess the growth of *L. plantarum* Inducia DSM 21379 and *C.difficile* reference strains by co-cultivation at different concentrations of xylitol and ampicillin.

The BHI media supplemented with 5% xylitol and 0.75 µl/ml ampicillin were applied for co-cultivation of *L. plantarum* Inducia DSM 21379 and *C. difficile* reference strains in anaerobic environment (workstation Concept 400, UK) for 24 and 48 h.

The viability of *L. plantarum* Inducia DSM 21379, *C.difficile* VPI and M reference strains after cocultivation were tested as single in BHI media with ampicillin and xylitol 5% (1, 2a, 2b). After ten-fold serial dilutions for determing the count of *L. plantarum* Inducia in MRS broth was cultivated in CO₂ environment and for *C. difficile* on LAB160 media in anaerobic milieu.

**Table 14. Inhibition of C. difficile reference strains with growth of L. plantarum Inducia DSM 21379 after co-cultivation in BHI with 5% xylitol and 0.75 µl/ml ampicillin after 0, 24, 48h in anaerobic environment**

| Strains /co-cultivation | | Growth (log, CFU/ml) | | |
|---|---|---|---|---|
| | | 0 h | 24 h | 48h |
| 1. *L. plantarum* Inducia | | 5.7 | 8.5 | 7.3 |
| 2. a. *C. difficile* VPI | | 5.0 | 7.7 | 7.2 |
| 2.b. *C. difficile* M reference strain | | 5.0 | 7.3 | 7.3 |
| 3. *L. plantarum* Inducia | *C. difficile* VPI | 6.0 | 8.3 | 7.0 |
| | M reference strain | 5.5 | 8.5 | 8.0 |
| 4. *C. difficile* VPI | *L. plantarum* Inducia | 5.0 | 0 | 0 |
| 5. M reference strain | | 5.0 | 5.0 | 2.0 |

During *in vitro* co-cultivation of *L. plantarum* Inducia DSM 21379 (3) and reference strains of *C. difficile* (4, 5) in the BHI medium with xylitol and ampicillin (mimicking the hamster model) the growth of vegetative cells of both *C. difficile* strains was suppressed. Full suppression was detectable for VPI strain and substantial reduction for 5 logarithms (from 5.0 to 2.0 log CFU/ml) was detected in the case of *C. difficile* strain M.

Thus, the *in vitro* experiments mimicking the gut environment of experimental hamster model after antibiotic treatment showed either full or high suppression of *C. difficile* growth with *L. plantarum* Inducia DSM 21379 alone (Table 5) or combined it with xylitol (Table 14).

### Example 8. Lactobacillus plantarum Inducia DSM 21379 and xylitol combination inhibit germination of spores of Clostridium difficile reference strain VPI.

The purpose of the experiment was to evaluate the ability of *L. plantarum* Inducia DSM 21379 to prevent *Clostridium difficile* germination, in the case of *L. plantarum* Inducia DSM 21379 preincubation in media. The effect of xylitol and taurocholate on the germination of *Clostridium difficile* spores was tested. Taurocholate is the natural agent in small intestine that promotes the germination of spores of *Clostridium difficile.*

*L. plantarum* Inducia DSM 21379 was incubated with various agents containing Brain Heart Infusion (BHI) broth in anaerobic condition for 24h. After incubation of *L. plantarum* Inducia DSM 21379, the *Clostridium difficile* spores were added to the mixture, and concentrated BHI broth in ratio 1:10.

The experimental and control mixture was incubated up to 48 hours in anaerobic condition. The mictures were seeded on Fastiodious Anaerobe Agar and Man Rogosa Sharpe Agar at the beginning of the experiment, after 24 and 48 hours. The count of *L. plantarum* Inducia DSM 21379and *Clostridium difficile* was calculated.

**Table 15. Clostridium difficile counts (log₁₀) at the presence of different factors in growth environment (L. plantarum Inducia DSM 21379, xylitol, taurocholate).**

| Mixture | Control (log10 CFU/ml) | | | Experimental, *L. plantarum* Inducia DSM 21379 was added (log10 CFU/ml) | | |
|---|---|---|---|---|---|---|
| | 0 h | 24 h | 48 h | 0 h | 24 h | 48 h |
| BHI | 2.95 | 6.05 | 7.45 | nd | nd | 7.18 |
| BHI + xylitol | 2.94 | 7.37 | 7.95 | nd | nd | nd |
| BHI + xylitol + sodium taurocholate hydrate | 3.83 | 7.83 | 7.96 | nd | nd | nd |
| BHI + xylitol + taurodeoxycholic acid | 3.38 | 8.07 | 7.67 | nd | nd | nd |

| | | | | | | |
|---|---|---|---|---|---|---|
| nd- not detectable (below the determination limit, 1.00 log₁₀ CFU/ml) | | | | | | |

Preincubation of *L. plantarum* Inducia DSM 21379 in the media containing xylitol or combination of xylitol with taurodeoxycholic acid, inhibits *Clostridium difficile* germination (Table 15). *L. plantarum* Inducia DSM 21379 was not affected by the presence of xylitol and/or taurodeoxycholic acid. These results support the results of animal experiment.

### Example 9. Animal experiment

The purpose of the study was to assess the influence of *L. plantarum* Inducia DSM 21379 on *Clostridium difficile* reference strain VPI 10463 caused infection in the intestinal tract of *C. difficile* spores challenged hamsters.

Syrian hamsters *(Mesocricetus auratus)* provide a well-characterized model of *Clostridium difficile* infection. The colonic microbiota of hamsters treated with antibiotics is disrupted and if afterwards exposed to *C. difficile* spores, the animals develop *C. difficile* associated diarrhea (CDAD) in less than 48 h with 100% of mortality.

We tested if mortality of *C. difficile* challenged hamsters can be decreased by administration *of L. plantarum* Inducia DSM 21379 according to our *in vitro* results. First, we pre-feeded hamsters daily with *L. plantarum* Inducia DSM 21379 in the concentration of 10¹⁰ cfu/ml by gastric gavage with/without 1 ml 20% xylitol for 5 consecutive days before administration of ampicillin (30 mg/kg). The administration of *L. plantarum* Inducia DSM 21379 in the aforementioned concentration was continued during the whole experiment. On Day 7 the hamsters were infected with 10-30 spores of *C. difficile* VPI 10463I. For next 5 days the health of hamsters was followed and in case of symptoms indicating CDAD: wet tail - the *C. difficile* A/B toxin test was performed (X/pect Remel test). The group of hamsters infected with *C. difficile* VPI strain served as a control group.

**Table 16. Survival of C. difficile challenged hamsters (n=15) pretreated with L. plantarum Inducia DSM 21379 and/or xylitol**

| Test groups | Number of survived hamsters after challenge with *C*. *difficile* | | | | | Toxin test result/number of animals in group | % of survival on Day 5 |
|---|---|---|---|---|---|---|---|
| | Day 1 | Day 2 | Day 3 | Day 4 | Day 5 | | |
| *C. difficile* VPI 10463I (n=15) | 15 | 6 | 4 | 2 | 2 | 2 negative/ 15 | 2/15 |
| | | | | | | | 13% |
| *C. difficile* VPI +Inducia (n=5) | 5 | 2 | 2 | 1 | 0 | 0 negative/5 | 0/5 |
| | | | | | | | 0% |
| *C. difficile* VPI +xylitol (n=9) | 9 | 9 | 9 | 5 | 5 | 4 negative/ 9 | 5/9 |
| | | | | | | | 56% |
| *C. difficile* VPI + xylitol + Inducia (n=9) | 9 | 7 | 7 | 7 | 7 | 7 negative/ 9 | 7/9 |
| | | | | | | | 78% |

Thus, the highest survival was found in case of combining *L. plantarum* Inducia DSM 21379 with xylitol solution pre-feeding prior to challenge with *C. difficile* spores (Table 16). In this case the toxin tests were all negative in the survived hamsters (7/9), i.e. that the germination of spores *C. difficile* was suppressed. If xylitol alone was applied, the toxin tests of 4 out of 5 surviving animals were negative and only in one surviving animal the toxin test was positive.

Thus, the combination of xylitol with *L. plantarum* Inducia DSM 21379 works through suppression of germination of spores into vegetative cells of *C. difficile* able of toxin producing.

We tested if the growth of *C. difficile* was still present after the intervention with Inducia and xylitol and if the A and B toxins of *C. difficile* were found (Table 17). In survived hamsters prefeeded with combination of *L. plantarum* Inducia DSM 21379 and xylitol the *C. difficile* vegetative cells were not found 6/7 cases (only in one case the *C. difficile* growth was seen) in jejunum and ileum. Both the counts of anaerobes and *L*. *plantarum* Inducia DSM 21379 substituting indigenous lactobacilli in jejunum and ileum were high showing the reconstruction and stabilisation of intestinal microbiota after administration of ampicillin, challenge with *C. difficile* spores and usage of the treatment with *L. plantarum* Inducia DSM 21379 and xylitol.

**Table 17. Total counts of anaerobes, lactobacilli, L. plantarum Inducia DSM 21379 and C. difficile in jejunum and ileum of survived hamster prefeeded with xylitol and L. plantarum Inducia DSM 21379**

| Treatment groups | Hamster ID | jejunum (CFU log10/g) | | | ileum (CFU log10/g) | | |
|---|---|---|---|---|---|---|---|
| | | AN | Inducia | *C.d.* | AN | Inducia | *C.d.* |
| *C. difficile* VPI + xylitol + Inducia (n=7) | T2-1 | 6.48 | 4.00 | 0.00 | 7.48 | 6.70 | 0.00 |
| | T2-2 | 5.20 | 4.30 | 0.00 | 7.04 | 7.00 | 0.00 |
| | T2-3 | 7.48 | 4.30 | 0.00 | 7.30 | 6.70 | 0.00 |
| | T2-4 | 8.70 | 6.00 | 5.00 | 8.11 | 7.60 | 7.00 |
| | T2-5 | 4.48 | 0.00 | 0.00 | 7.30 | 6.00 | 0.00 |
| | T2-7 | 7.18 | 5.00 | 0.00 | 8.11 | 6.78 | 0.00 |
| | T2-8 | 8.34 | 6.00 | 0.00 | 8.43 | 6.00 | 0.00 |
| *C. difficile* VPI + xylitol (n=5) Pre-feeded | Xyl-1 | 5.30 | ND | 2.00 | 8.43 | ND | 0.00 |
| | Xyl-2 | 7.11 | ND | 0.00 | 8.30 | ND | 0.00 |
| | Xyl-3 | 7.60 | ND | 5.00 | 8.38 | ND | 7.60 |
| | 24-1 | 7.35 | ND | 0 | 7.0 | ND | 0 |
| | 24-2 | 7.15 | ND | 0 | 7.8 | ND | 0 |

| | | | | | | | |
|---|---|---|---|---|---|---|---|
| AN - anaerobes; *C.d.* - *Clostridium difficile* | | | | | | | |

The survival rate of hamsters was 22 % higher due to *L. plantarum* Inducia DSM 21379 (Table 16). This was proved also by absence of toxin from intestinal content of large intestine (Table 17).

The growth of *L. plantarum* Inducia DSM 21379 was high in jejunum (range 0 - 6.0, median 4.0 CFU log10/g and in ileum 6.0-7.6, median 6.7). *L. plantarum* Inducia DSM 21379 acted seemingly via its acid production through its SCFAs profile.

### Morphological evaluation of the C. difficile infected hamsters

The typical CDAD infection was modelled in hamster model as in *C.difficile* infection the toxin damage of organs is the leading pathogenetic modulator.

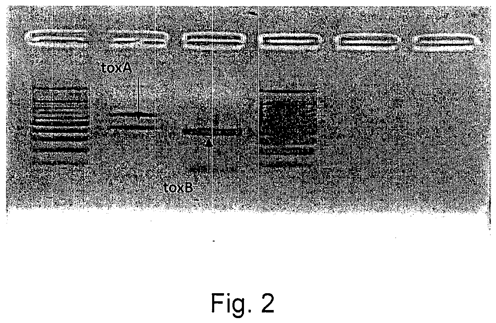

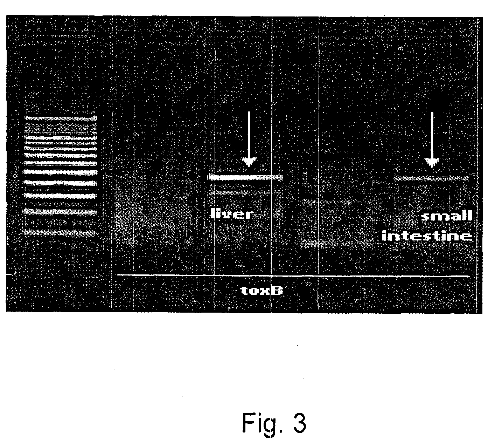

In our study the *C*. *difficile* VPI 10643 strain possessed both toxins A and B, and these were also present in liver and small intestine (Fig. 2 and 3).

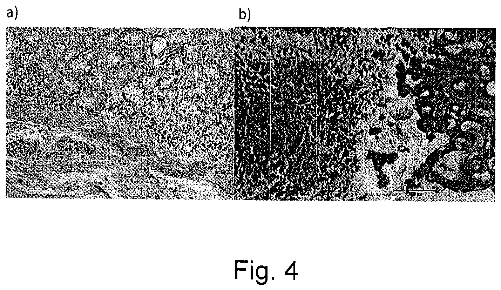

In hamsters surviving the *C*. *difficile* infection and examined during autopsy no extensive damage of mucosa, no pseudomembranes and severe infiltration with polymorphonuclear cells was seen (Fig 4 a).

Some hours before death the characteristic morphological finding of damaged with *C. difficile* infection hamsters was the inflammation with polymorphonuclear infiltration in mucosa and presence of pseudomembranes.

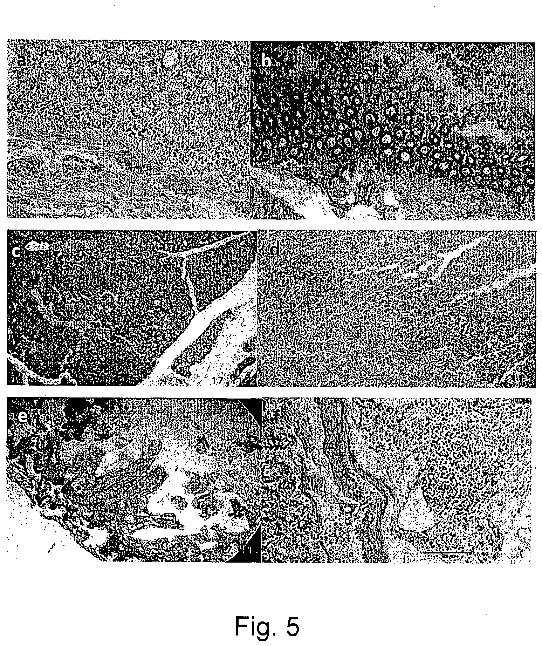

The severe enterocolitis developed the infiltration with red blood cells and polymorphonuclear leukocytes into gut mucosa, liver and spleen resulted in death of animals. In organs the hyperemia was present (Fig 5 a-f).

## Claims

1. A composition comprising the probiotic microorganism *Lactobacillus plantarum* Inducia strain DSM 21379 for use in preventing the cholesterol metabolism disorders and consecutive cardiovascular disorders by reducing the level of LDL-cholesterol in blood.

2. The composition for use according to claim 1, wherein said composition is a pharmaceutical composition, a food product, a food supplement or a veterinary composition.

3. The composition for use according to claim 2, wherein the food product is a dairy product.

4. The composition for use according to claim 2 or 3, wherein the food product is a fermented milk product.

5. The composition for use according to claim 2, 3 or 4, wherein the food product is cheese.

6. The composition for use according to claim 2, 3 or 4, wherein the food product is yoghurt.

7. *Lactobacillus plantarum* Inducia DSM 21379 for use as hypocholesterolemic agent in preventing the cholesterol metabolism disorders and consecutive cardiovascular disorders by decreasing the level of LDL-cholesterol in blood.

## Patentansprüche

1. Zusammensetzung, die das Mikroorganismenstamm *Lactobacillus plantarum* Inducia DSM 21379 enthält, die bei der Vorbeugung von Störungen im Cholesterinstoffwechsel und konsekutiven Herz-Kreislauferkrankungen durch die Reduzierung des LDL-Cholesterinspiegels im Blut verwendet wird.

2. Zusammensetzung zur Verwendung nach Anspruch 1, wobei die genannte Zusammensetzung eine pharmazeutische Zusammensetzung, ein Nahrungsprodukt, ein Nahrungsergänzungsmittel oder eine tierärztliche Zusammensetzung ist.

3. Zusammensetzung zur Verwendung nach Anspruch 2, wobei das Nahrungsprodukt ein Milchprodukt ist.

4. Zusammensetzung zur Verwendung nach Anspruch 2 oder 3, wobei das Nahrungsprodukt ein fermentiertes Milchprodukt ist.

5. Zusammensetzung zur Verwendung nach Anspruch 2, 3 oder 4, wobei das Nahrungsprodukt Käse ist.

6. Zusammensetzung zur Verwendung nach Anspruch 2, 3 oder 4, wobei das Nahrungsprodukt Joghurt ist.

7. *Lactobacillus plantarum* Inducia DSM 21379 zur Verwendung als hypocholesterolemischen Wirkstoff bei der Vorbeugung von Störungen im Cholesterinstoffwechsel und konsekutiven Herz-Kreislauferkrankungen durch die Reduzierung des LDL-Cholesterinspiegels im Blut.

## Revendications

1. La composition comprenant le micro-organisme probiotique, la souche Inducia DSM 21379 de *Lactobacillus plantarum* pour l'utilisation dans la prévention des troubles du métabolisme du cholestérol et des troubles cardiovasculaires consécutifs en réduisant le taux du cholestérol LDL dans le sang.

2. La composition pour l'utilisation selon la revendication 1, dans laquelle ladite composition est une composition pharmaceutique, un produit alimentaire, un complément alimentaire ou une composition vétérinaire.

3. La composition pour l'utilisation selon la revendication 2, dans laquelle le produit alimentaire est un produit laitier.

4. La composition pour l'utilisation selon les revendications 2 ou 3, dans laquelle le produit alimentaire est un produit laitier fermenté.

5. La composition pour l'utilisation selon les revendications 2, 3 ou 4, dans laquelle le produit alimentaire est du fromage.

6. La composition pour l'utilisation selon les revendications 2, 3 ou 4, dans laquelle le produit alimentaire est un yaourt.

7. La souche Inducia DSM 21379 de *Lactobacillus plantarum* pour l'utilisation en tant qu'agent hypocholestérolémique dans la prévention des troubles du métabolisme du cholestérol et des troubles cardiovasculaires consécutifs en diminuant le taux de cholestérol LDL dans le sang.
